# EUROPEAN PATENT SPECIFICATION

(11) **EP 3 547 990 B1**
(45) Date of publication and mention of the grant of the patent: **19.02.2025**
(21) Application number: 17825125.2
(22) Date of filing: 30.11.2017
(51) Int. Cl.: A61Q 17/04, A61K 8/73

(54) **SUNSCREEN COMPOSITION COMPRISING NANOCRYSTALLINE CELLULOSE**
SONNENSCHUTZZUSAMMENSETZUNG MIT NANOKRISTALLINER CELLULOSE
COMPOSITION D'ÉCRAN SOLAIRE COMPRENANT DE LA CELLULOSE NANOCRISTALLINE

(30) Priority: 30.11.2016 US 201662427910 P
(43) Date of publication of application: 09.10.2019
(73) Proprietor: Anomera Inc., Montreal, QC H3B 1A7 (CA)
(72) Inventor: GUARILLOFF, Philippe, Piscataway, NJ 08854-3917 (US)
(74) Representative: Plasseraud IP
(86) International application number: PCT/EP2017/080983
(87) International publication number: WO 2018/100062

(56) References cited:
- WO-A1-2013/033833
- WO-A1-2016/015148
- CN-A- 103 230 355
- CN-A- 106 109 329
- US-A1- 2007 104 662

## Description

### Technical field

The present invention relates to fluid compositions for protecting the skin from the effects of ultraviolet radiations, hereinafter designated as "sunscreen" or "photoprotective" compositions. This invention relates more especially to sunscreen compositions with improved properties, namely homogeneity, fluidity and transparency on the skin.

### Background of the invention

### - related prior art

It is well known that ultraviolet radiation from the sun can have damaging effects on the skin and the hair. The wavelengths from 210 to 280 nm are stopped by the ozone layer, they are known as UV-C rays. The wavelengths from 280 to 400 nm pass through the ozone layer. Between 280 and 320 nm the rays are known as UV-B, whereas UV-A rays relate to the wavelengths between 320 and 400 nm.

UV-A and UV-B correspond to the rays that allow the tanning of the skin. However, when the exposure is too important or the skin too sensitive, these rays may induce impairments of the skin, such as skin burns or erythema. At medium and long term, they may also induce a premature aging by promoting a loss of skin elasticity and the appearance of wrinkles. In more severe cases, they may lead to skin cancers.

It is thus desirable to screen out both UV-A and UV-B to protect the skin from these damages.

To date, many photoprotective cosmetic compositions have been proposed to screen out UV-A and/or UV-B, but there is still an increasing demand for very fluid sunscreen compositions having good cosmetic properties and providing a high protection against UV radiations.

The protection intensity provided by a sunscreen composition is usually expressed by the sun protection factor or SPF. It is a measure of the dose of UV radiation required to produce sunburn on protected skin, i.e. on which a sunscreen has been applied, relative to the dose of radiation required to produce sunburn on unprotected skin.

### - Problem with prior art:

Photoprotective compositions usually comprise at least one ultraviolet screening agent.

Said at least one ultraviolet screening agent may be chosen from lipophilic and/or hydrophilic organic compounds capable of selectively absorbing wavelengths. The degree of UV protection afforded by a composition is directly related to the amount and type of sunscreen agents present: the higher the concentration, the greater the protection. Besides, the combination of several UV screening agents makes it possible to cover the wider range of wavelengths. Thus, to reach high SPF compositions, it is usually necessary to add large quantities of several organic screening agents.

However, when the amounts of these organic compounds are too important, the composition loses its cosmetic qualities. It may result in impairments of comfort and cause a greasy and/or tacky feeling.

### - Solutions from the prior art

One solution known from the prior art to counterbalance this oily effect consists in using mineral sunscreens, either in combination with organic sunscreen, or alone. Mineral sunscreens such as titanium dioxide or zinc oxide are well appreciated because they allow to efficiently reaching a high SPF without producing the above-mentioned drawbacks of organic sunscreens.

However, mineral sunscreens are characterized by a high refractive index, which produces a whitening effect on the skin during application. Also, when they are present in large amounts, mineral sunscreen may agglomerate and create clusters in the composition. This phenomenon accentuates the whitening effect of the compositions and may leave residues on the skin. Moreover, the viscosity of the composition rises when the concentration of mineral sunscreen increases.

As a consequence, the use of mineral filters in photoprotective compositions does not allow formulators to add other powders, such as texture enhancing or optical effect particles in order to enhance the skin feel or produce an optical immediate effect, , because they would accentuate these phenomena. CN103230355 discloses sunscreen cream cosmetic composition containing nano crystal cellulose. WO2013033833 discloses a cosmetic composition with enhanced photoprotection properties. CN106109329 discloses nano sunscreen gel compositions. WO2016015148 relates to a method for producing functionalized nanocrystalline cellulose and to the functionalized nanocrystalline cellulose produced by this method. US2007104662 relates to an ultraviolet-absorbing composite powder comprising a plurality of composite powder particles

### - unfulfilled need

Thus, the use of mineral UV screening agents implies many limitations in terms of formulation. Thereby, there remains a need for having photoprotective compositions with a high SPF exhibiting a good fluidity and homogeneity, with limited whitening effect on the skin.

### Aims and advantages of the invention

The inventors have discovered that the incorporation of functionalized nanocrystalline cellulose as defined in claim 1, also referred to as NCC, said functionalized nanocrystalline cellulose being carboxylated nanocrystalline cellulose, in photoprotective compositions allows to overcome the abovementioned drawbacks of the prior art. The inclusion of carboxylated nanocrystalline cellulose does not thicken the compositions according to the invention. It does not alter the transparency, and allows improving the homogeneity of compositions comprising mineral filters.

Thus, one aim of the present invention is to provide sunscreen compositions with satisfactory photoprotective properties.

Another aim of the invention is to provide fluid serum-type sunscreen compositions without drop or increase of the viscosity over time.

Another aim of the invention is to provide sunscreen compositions with satisfactory transparency of the film applied on the skin, causing very low whitening and thus being not detectable to human eye. Another aim of the invention is to provide a sunscreen composition with a good homogeneity.

The term "fluid composition" means a composition that is not in a solid form at room temperature (25°C) and atmospheric pressure (1,013.10⁵ Pa).

### Summary of the invention

The present invention is according to the claims and therefore directed to a sunscreen composition, in a physiologically acceptable medium, comprising at least one UV screening agent and functionalized nanocrystalline cellulose, wherein said functionalized nanocrystalline cellulose is carboxylated nanocrystalline cellulose.

Another object of the invention is to provide a sunscreen composition comprising carboxylated nanocrystalline cellulose, wherein said carboxylated nanocrystalline cellulose is in the form of a nanocrystalline cellulose carboxylate salt.

Another object of the invention is to provide a sunscreen composition comprising nanocrystalline cellulose carboxylate salt, wherein said nanocrystalline cellulose carboxylate salt is a nanocrystalline cellulose sodium carboxylate.

Another object of the invention is to provide a sunscreen composition comprising nanocrystalline cellulose sodium carboxylate, wherein said nanocrystalline cellulose sodium carboxylate is produced by the method comprising the steps of:
a) providing cellulose,
b) mixing said cellulose with a peroxide, thereby producing a reaction mixture,
c) heating the reaction mixture, and/or exposing the reaction mixture to UV radiation, and
d) salifying the reaction mixture.

Another object of the invention is to provide a sunscreen composition comprising carboxylated nanocrystalline cellulose, wherein said nanocrystalline cellulose has a spherical or ovoid shape.

Another object of the invention is to provide a sunscreen composition comprising carboxylated nanocrystalline cellulose, wherein said nanocrystalline cellulose has an average particle size of less than about 20 µm, preferably from 2µm to 10µm. The average particle size is the particle size distribution D50, also known as the median diameter or the medium value of the particle size distribution, it is the value of the particle diameter at 50% in the cumulative distribution. The particle size distribution is determined by Scanning Electron Microscopy (SEM).

Another object of the invention is to provide a sunscreen composition comprising carboxylated nanocrystalline cellulose, wherein said nanocrystalline cellulose has an oil uptake of less than 70 mL for 100 g of nanocrystalline cellulose.

Another object of the invention is to provide a sunscreen composition comprising carboxylated nanocrystalline cellulose, wherein said nanocrystalline cellulose has an oil uptake of from 35 to 65 mL for 100 g of nanocrystalline cellulose, more preferably from 40 to 60 mL for 100 g of nanocrystalline cellulose.

Another object of the invention is to provide a sunscreen composition comprising carboxylated nanocrystalline cellulose, wherein said nanocrystalline cellulose is present in an amount of from about 0.1% to about 50%, preferably from 0.2% to 30%, more preferably from 0.5% to 10%.

Another object of the invention is to provide a sunscreen composition comprising carboxylated nanocrystalline cellulose and at least one sunscreen agent, wherein the at least one sunscreen agent is selected from mineral sunscreen agents and/or organic sunscreen agents.

Another object of the invention is to provide a sunscreen composition comprising carboxylated nanocrystalline cellulose and at least mineral sunscreen agent, wherein the at least mineral sunscreen agent is selected from zinc oxide and/or titanium dioxide.

Another object of the invention is to provide a sunscreen composition comprising carboxylated nanocrystalline cellulose and comprising a water phase and an oily phase.

Another object of the invention is to provide a sunscreen composition comprising carboxylated nanocrystalline cellulose, wherein said sunscreen composition comprises at least one surfactant.

Another object of the invention is to provide a sunscreen composition comprising carboxylated nanocrystalline cellulose, wherein said sunscreen composition is surfactant-free.

Another object of the invention is to provide a sunscreen composition comprising carboxylated nanocrystalline cellulose, wherein said composition comprises at least one humectant.

Another object of the invention is to provide a sunscreen composition comprising carboxylated nanocrystalline cellulose, wherein said composition comprises at least one emollient.

Another object of the invention is to provide a sunscreen composition comprising carboxylated nanocrystalline cellulose, wherein the photoprotection composition further comprises at least one ingredient chosen from preservatives, cosmetic active ingredients, moisturizers, alcohol and/or fragrances.

Another object of the invention is to provide a sunscreen composition comprising carboxylated nanocrystalline cellulose, wherein said sunscreen composition is in the form of a gel, a lotion, a serum.

Other characteristics, aspects and advantages of the present invention will become apparent on reading the detailed description which follows.

### Detailed description

The cosmetic compositions according to the present invention comprise nanocrystalline cellulose, also referred to as "NCC". Nanocrystalline cellulose is derived from native cellulose from which the amorphous part is removed to keep only the crystalline part.

According to the present invention, the amorphous part of native cellulose is advantageously removed by oxidative hydrolysis of native cellulose using a peroxide, such as hydrogen peroxide, an organic peroxide or a mixture thereof. This process of dissolution of the amorphous part of native cellulose using a peroxide produces nano-crystallites of cellulose, which are then assembled into larger particles corresponding to said nanocrystalline cellulose or NCC.

Said nanocrystalline cellulose is functionalized, i.e. it has undergone a surface modification to produce functionalized nanocrystalline cellulose. According the invention, said functionalized nanocrystalline cellulose is a carboxylated nanocrystalline cellulose.

Advantageously, carboxylated nanocrystalline cellulose may undergo total or partial salification to produce nanocrystalline cellulose carboxylate salt.

According to another embodiment, the nanocrystalline cellulose carboxylate salt according to the present invention is produced by the method comprising the steps of:
a) providing cellulose,
b) mixing said ellulose with a peroxide, thereby producing a reaction mixture,
c) heating the reaction mixture, and/or exposing the reaction mixture to UV radiation, and
d) salifying the reaction mixture.

According to another embodiment, the assemblage of nano-crystallites of cellulose into particles of nanocrystalline cellulose is achieved by spray-drying.

According to another embodiment, said particles of nanocrystalline cellulose have a spherical or ovoid shape, or a mixture thereof.

According to another embodiment, nanocrystalline cellulose has an average particle size of less than 20 µm, preferably less than 15 µm, more preferably between 2 µm and 10 µm.

According to another embodiment, nanocrystalline cellulose has an oil uptake of less than 70 mL/100g, preferably between 35 and 65 mL/100g, and more preferably between 40 and 60 mL/100g. The oil uptake characterizes the ability to adsorb castor oil. It is determined by adding castor oil to 100 g of nanocrystalline cellulose powder. The oil uptake corresponds to the minimal amount of castor oil, in milliliters, required to obtain a firm and homogeneous paste with 100 g of powder.

According to another embodiment, nanocrystalline cellulose has a contact angle with water between 80° and 100°, preferably between 85° and 95°, and more preferably between 88° and 92°.

Preferentially nanocrystalline cellulose used in the present invention is nanocrystalline cellulose obtained by the process described in the disclosure of patent application WO 2016/015148, incorporated herein by reference.

The compositions according to the inventions may comprise a water phase and an oily phase.

According to one embodiment, the compositions according to the inventions are in the form of an emulsion.

According to one embodiment, the compositions of the invention may comprise at least one surfactant. As emulsifying surfactants that may be used, mention may be made of sorbitan, glycerol or sugar alkyl esters or ethers, such as polyglyceryl isostearate, sorbitan isostearate, polysorbate-65; silicone surfactants such as dimethicone copolyol and alky dimethicone copolyol such as lauryl dimethicone.

According to one particular embodiment, the compositions of the invention are surfactant-free.

The compositions according to the invention comprise at least one UV screening agent which may be chosen from mineral and/or organic sunscreen agents.

Examples of mineral sunscreen agents include pigments and nanopigments formed from metal oxides. Among metal oxides, mention may be made of titanium oxide, iron oxide, zinc oxide, zirconium oxide and cerium oxide, which are all well-known as UV photoprotective agents.

The mineral sunscreen agents may be surface coated.

Among the surface-coatings that may be used in the present invention, mention may be made of aluminium hydroxide; alumina; polyurethane derivatives; polyquaternium derivatives; silicone derivatives such as triethoxycaprilylsilane (OTS coating from Daito Kasei), triethoxysilylethyl polydimethylsiloxyethylhexyl dimethicone, acrylates/dimethicone copolymer (FSA coating from Daito Kasei), methicone or dimethicone; amino-acid derivatives or N-acylamino acids or salts thereof such as sodium lauroyl glutamate, sodium lauroyl aspartate, lysine, disodium stearoyl glutamate, lauroyl lysine; fluoro derivatives such as perfluoroalkylsilanes, perfluoroalkylsilazanes, perfluoroalkyl phosphates, C9-C15 fluoroalcohol phosphates; lecithin derivatives such as hydrogenated lecithin; alkyl titanated derivatives such as isopropyl titanium triisostearate; silica; silicates such as potassium aluminium silicate; fatty acid derivative such as stearic acid; metallic soaps such as aluminium dimyristate, aluminium stearate, magnesium myristate, metal oxides such as titanium dioxide, zinc oxide or iron oxide; and mixture thereof.

In a preferred embodiment, the compositions according to the invention comprise nanopigments of titanium dioxide and/or zinc oxide.

Examples of organic sunscreens include dibenzoylmethane derivatives; cinnamic acid derivatives; salicylates derivatives; para-aminobenzoic acids; β,β'-diphenylacrylate derivatives; benzophenone derivatives; benzylidenecamphor derivatives; phenylbenzimidazole derivatives; triazine derivatives; phenylbenzotriazole derivatives; anthranilic acid derivatives, and mixtures thereof. All of them may be encapsulated.

Non-limiting examples of organic filters that may be used in the present invention include those having the INCI names Benzophenone-1, Benzophenone-2, Benzophenone-3, Benzophenone-4, Benzophenone-5, Benzophenone-6, Benzophenone-8, Benzophenone-9, butyl methoxydibenzoylmethane (commercially available from HOFFMANN LA ROCHE under the trade name of Parsol 1789), octyl methoxycinnamate (commercially available from HOFFMANN LA ROCHE under the trade name of Parsol MCX), cinoxate, terephtalylidene dicamphor sulphonic acid, 3-benzylidene Camphor, Camphor Benzalkonium Methosulfate, Bis-Ethylhexyloxyphenol Methoxyphenyl Triazine, Diethylamino Hydroxybenzoyl Hexyl Benzoate, Diisopropyl Methyl Cinnamate, 1-(3,4-Dimethoxyphenyl)-4,4-Dimethyl-1,3-Pentanediene, Disodium Phenyl Dibenzimidazole Tetrasulfonate, Drometrizole trisiloxane, Ethylhexyl Dimethoxybenzylidene Dioxoimidazolidine Propionate, Ethylhexyl Dimethyl PABA, Ethylhexyl Methoxycinnamate, Ethylhexyl Salicylate, Ethylhexyl Triazone, Ferulic Acid, 4-(2-Beta-Glucopyranosiloxy) Propoxy-2-Hydroxybenzophenone, Glyceryl Ethylhexanoate Dimethoxycinnamate, Homosalate, Isoamyl p-Methoxycinnamate, Isopentyyl Trimethoxycinnamate Trisiloxane, Isopropyl Methoxycinnamate,
Menthyl Anthranilate, 4-Methylbenzylidene Camphor, Methylene Bis-Benzotriazolyl Tetramethylbutylphenol, Octocrylene, PABA, PEG-25 PABA, Pentyl Dimethyl PABA, Phenylbenzimidazole Sulfonic Acid and its salts, Polyacrylamidomethyl Benzylidene Camphor, Polysilicone-15, Potassium Phenylbenzimidazole Sulfonate, Sodium Phenylbenzimidazole Sulfonate, TEA-Phenylbenzimidazole Sulfonate, TEA Salicylate, Terephthalylidene Dicamphor Sulfonic Acid, and mixtures thereof.

The compositions according to the inventions may also comprise at least one humectant. Non-limiting example include glycerol derivatives such as glycerin, butylene glycol, propylene glycol, caprylyl glycol; urea derivatives; lactic acid derivatives.

The compositions according to the invention may comprise at least one emollient which may be chosen from liquid oils.

The term "liquid" refers to compounds in a liquid state at room temperature (i.e. 20°C) and atmospheric pressure (i.e. 1.013×10⁵ Pa).

The term "oil" refers to any compound that is not miscible in water and which is liquid at room temperature (i.e. 20°C) and atmospheric pressure (i.e. 1.013×10⁵ Pa).

The liquid binder phase advantageously includes at least one non-volatile oil, which may be hydrocarbon-based oil, silicone-based oil or a mixture thereof. The oils according to the invention may be synthetic or from natural origin.

The term "non-volatile oil" is understood to mean any liquid oil which is not capable of evaporating on contact with the skin, and thus remaining on the skin.

The term "hydrocarbon-based oils" means oils mainly containing carbon atoms and hydrogen atoms, and which may also comprise one or more functional group selected from alcohol, ether, ester, fluoro and/or carboxylic acid groups.

The term "silicone-based oils" means oils containing silicon atoms but also oxygen, carbon and hydrogen atoms. Silicone-based oils may also comprise one or more functional group such as alcohol, ether, ester, fluoro and/or carboxylic acid groups.
- Silicone-based oils include but are not limited to linear and cyclic non-volatile polydimethylsiloxanes, polymethylphenylsiloxanes, phenyl dimethicones, phenyl trimethicones; polysiloxanes modified with fatty acids fatty alcohols, alkylene oxyalkylene groups or, amine group; fluorosilicones or perfluoro silicone oils;
- Hydrocarbon-based oils include hydrocarbon oils, esters of fatty acids, fatty alcohols, fatty acids and/or vegetable oils.
- Hydrocarbon oils which may be linear or branched, saturated or unsaturated, such as liquid paraffins, mineral oil, squalane, squalene, polydecenes, polybutenes and derivatives;
- Esters of fatty acids of general formula R1COOR2 wherein R1 represents a linear or branched fatty acid residue containing from 1 to 40 carbon atoms, preferably from 1 to 30 carbon atoms, more preferably from 1 to 22 carbon atoms, and R2 represents a hydrocarbon-based chain which may be linear or branched too, and containing from 1 to 40 carbon atoms. These two carbon chains may be saturated or unsaturated. The esters may also contain a polyalkylene glycol branching such as polypropylene glycol or polyethylene glycol branching, for example PPG-2 myristyl ether propionate. The compositions according to the invention may also comprise polyesters, i.e. compounds comprising more than one ester functional group such as diesters or triesters. Mention may be made of triglycerides formed by esterification of glycerol such as caprylic/capric triglyceride; esters of polyglycerin such as polyglyceryl-2 triisostearate; triethylhexanoin, dicaprylyl carbonate or octyldodecyl stearoyl stearate. The acid residue may also be cyclic, such as in esters of benzoic acid or esters of salicylic acid.

Suitable fatty acid esters include without limitation isononyl isononanoate, isopropyl myristate, 2 ethylhexyl palmitate, hexyl laurate, diisostearyl malate, C12-15 Alkyl Ethylhexanoate, cetyl ethylhexanoate, octyl stearate, isodecyl neopentanoate, isostearyl palmitate, alkyl benzoates, butyl acetate, butyl lsostearale, butyl oleate, butyl octyl oleate, cetyl palmilale, ceyl oclanoale, celyl laurate, cetyl lactate, cetyl isononanoate, cetyl stearate, diisostearyl fumarate, diisostearyl malale, neopentyl glycol dioctanoate, dibutyl sebacate, di-C12-13 alkyl malate, dicetearyl dimer dihnoleate, dicetyl adipate, dusocetyl adipate, dusononyl adipate, dusopropyl dunerate, triisostearyl trihnoleate, octodecyl stearoyl stearate, hexyl laurate, hexadecyl isostearate, hexydecyl laurate, hexyldecyl octanoate, hexyldecyl oleate, hexyldecyl palmitate, hexyldecyl stearate, isononyl isononanaote, isostearyl lsononate, isohexyl neopentanoate, isohexadecyl stearate, isopropyl isostearate, n-propyl myristate, isopropyl mynstate, n-propyl palmitate, isopropyl palmitate, hexacosanyl palmitate, lauryl lactate, octacosanyl palmitate, propylene glycol monolaurate, triacontanyl palmitate, dotriacontanyl palmitate, tetratriacontanyl palmitate, hexacosanyl stearate, octacosanyl stearate, triacontanyl stearate, dotriacontanyl stearate, stearyl lactate, stearyl octanoate, stearyl heptanoate, stearyl stearate, tetratriacontanyl stearate, triarachidin, tributyl citrate, triisostearyl citrate, tri-C[12-13]-alkyl citrate, tricapryhn, tricaprylyl citrate, tridecyl behenate, trioctyldodecyl citrate, tridecyl cocoate, tridecyl isononanoate, glyceryl monoricinoleate, 2-octyldecyl palmitate, 2-octyldodecyl myristate or lactate, di(2-ethylhexyl) succinate, tocopheryl acetate, tripropylene glycol dineopentanoate, cetyl octanoate, cetyl isooctanoate, octyldodecyl myristate, isopropyl palmitate, cetyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprylate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, glyceryl trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, bis-behenyl/isostearyl/phytosteryl dimer dilinoleyl dimer dilinoleate, phytosteryl/behenyl/octyldodecyl/isostearyl lauroyl glutamate, caprylic/capric triglyceride, and triethylhexanoin.
- Fatty alcohols, preferably having from 5 to 40 carbon atoms such as octyldodecanol and oleyl alcohol.
- Fatty acids preferably having from 5 to 40 carbon atoms such as linoleic or linolenic acid.
- Vegetable oils and derivatives, such as soybean oil, jojoba oil, olive oil, macadamia oil, liquid butyrospermum parkii (shea butter), castor oil, camellia oil, gardenia oil, avocado oil, coconut oil, argania spinosa kernel oil, corn oil, cottonseed oil, linseed oil, mink oil, soybean oil, grape seed oil, sesame oil, maize oil, rapeseed oil, sunflower oil, peanut oil, teas seed oil, rice bran oil.
- Sarcosine derivatives such as isopropyl lauroyl sarcosinate.

The composition advantageously comprises at least one volatile oil.

The cosmetic oils that are volatile at ambient temperature especially have a vapour pressure, measured at ambient temperature and atmospheric pressure, ranging from 10-3 mmHg to 300 mmHg (0.266 Pa to 40 000 Pa).

As a volatile oil that can be used in the invention, mention may be made of linear or cyclic silicones oils that have a viscosity at ambient temperature of less than 8 cSt and that especially have from 2 to 7 silicon atoms, these silicones optionally comprising alkyl or alkoxy groups having from 1 to 10 carbon atoms. As a volatile silicone oil that can be used in the invention, mention may especially be made of octamethylcyclotetrasiloxane, decamethylcyclopentasiloxane, dodecamethylcyclo-hexa-siloxane, heptamethylhexyltrisiloxane, heptamethyl-octyl-trisiloxane, hexamethyldisiloxane, octamethyltrisiloxane, decamethyltetrasiloxane, dodecamethylpentasiloxane and mixtures thereof.

As another volatile oil that can be used in the invention, mention may be made of volatile hydrocarbon-based oils having from 8 to 16 carbon atoms and mixtures thereof, and especially C8-C16 branched alkanes such as C8-C16 isoalkanes (also called isoparaffins), isododecane, isodecane, isohexadecane and, linear volatile alkanes from 9 to 14 carbon atoms, such as those sold under the names Vegelight 1214 from Biosynthis and Cetiol Ultimate from BASF.

The compositions according to the present invention may also comprise additional ingredients usually used in cosmetics, such as preserving agents, cosmetic active ingredients, moisturizers, and/or fragrances. The preserving agents that may be used include for example Ammonium silver zinc aluminium silicate, chlorophenesin, potassium sorbate, sodium dehydroacetate, and mixture thereof.

Among the cosmetic active ingredients that may be used in the present invention, mention may be made of whitening agents, brightening agents, antioxidant agents, anti-wrinkles agents, antiseborrheic agents, plant extracts, and mixture thereof.

Non-limiting examples of active ingredients include hyaluronic acid derivatives such as sodium hyaluronate, vitamin derivatives, such as tocopheryl actetate, ascorbic acid derivatives such as ascorbyl glucoside, Niacinamide, Licorice extract, Kalanchoe Pinnata leaf extract, Vanilla Planifolia extract.

### Tests of stability and viscosity

The examples which follow are used to illustrate the invention without however presenting a restrictive character. In these examples, the quantities of ingredients are given in weight percentage compared to the total weight of the composition.

A series of three sunscreen compositions in the form of emulsions have been prepared in order to follow the stability and viscosity. The compositions are given in table 1.

**Table 1: compositions of the three emulsions**

| Ingredients | Composition n° | | |
|---|---|---|---|
| | 1 | 2 | 3 |
| Zinc Oxide | 18% | 18% | 18% |
| Polyglycerol-6 polyricinoleate | 5% | 5% | 5% |
| C12-15 Alkyl benzoate | 29% | 29% | 29% |
| Ethylhexyl methoxycinnamate | 6.8% | 6.8% | 6.8% |
| Ethylhexyl salicylate | 5% | 5% | 5% |
| Trimethylpentandiol/adipic acid/glycerin crosspolymer | 1% | 1% | 1% |
| Neopentylglycol diheptanoate / Propylene Glycol Dibenzoate | 1% | 1% | 1% |
| Water | 22.4% | 23.8% | 22.4% |
| Phenylbenzimidazol Sulfonic Acid | 2% | 2% | 2% |
| Sodium Hydroxide solution (25%w/w) | 1 % | 1.1% | 1% |
| **Nanocrystalline Cellulose** | 1.5% | - | - |
| Cellulose (Cellulobeads D-5) | - | - | 1.5% |
| Caprylyl glycol | 1% | 1% | 1% |
| Alcohol | 6% | 6% | 6% |
| Fragrance | 0.3% | 0.3% | 0.3% |

The stability has been visually evaluated after centrifugation the day after formulating the compositions, and then after spending one month at 45°C.

The viscosities have been measured one day after formulating the compositions with a Brookfield Viscometer using a S02 spindle. The viscosity values in centipoise (cPs) as well as the following parameters: percentage torque, temperature and the speed of the spindle, are given in table 2.

| Composition n° | Centrifugation after 1 day | After 1 month at 45°C | Viscosity after 1 day in cPs (%torque, temperature, speed) |
|---|---|---|---|
| 1 | stable | stable | 356 cPs (53.4%, 23.2°C, 60 RPM) |
| 2 | stable | stable but unsmooth aspect | 457.6 cP (57.2%, 22.3°C, 50 RPM) |
| 3 | stable but slight dephasing | stable | 428 cP (53.5%, 22.3°C, 50RPM) |

The three compositions were stable, although nanocrystalline cellulose gave the most stable composition both after centrifugation and at 45°C during 1 month.

Surprisingly, the inventors have found that the composition comprising nanocrystalline cellulose makes it possible to obtain the most fluid composition.

Moreover, totally unexpectedly, the addition of nanocrystalline cellulose made it possible to obtain a significantly lower viscosity than without the addition of additional powder, corresponding to composition n°2.

## Claims

1. A sunscreen composition comprising, in a physiologically acceptable medium,
(i) at least one sunscreen agent, and
(ii) a functionalized nanocrystalline cellulose,
wherein said functionalized nanocrystalline cellulose is carboxylated nanocrystalline cellulose,
wherein said functionalized nanocrystalline cellulose is made of nano-crystallites of cellulose assembled into particles of nanocrystalline cellulose,
wherein said particles have a spherical or ovoid shape, and
wherein said functionalized nanocrystalline cellulose has an average particle size from 2 µm to 10 µm.

2. The sunscreen composition according to claim 1 wherein said carboxylated nanocrystalline cellulose is in the form of a nanocrystalline cellulose carboxylate salt.

3. The sunscreen composition according to claim 1 wherein said nanocrystalline cellulose carboxylate salt is a nanocrystalline cellulose sodium carboxylate.

4. The sunscreen composition according to claim 2 wherein said nanocrystalline cellulose sodium carboxylate is produced by the method comprising the steps of:
(a) providing cellulose, and
(b) mixing said cellulose with a peroxide, thereby producing a reaction mixture, and
(c) heating the reaction mixture, and/or
(c') exposing the reaction mixture to UV radiation, and
(d) salifying the reaction mixture.

5. The sunscreen composition according to any of the preceding claims, wherein said functionalized nanocrystalline cellulose has an oil uptake of less than 70 mL for 100 g of nanocrystalline cellulose.

6. The sunscreen composition according to any of the preceding claims, wherein said functionalized nanocrystalline cellulose has an oil uptake of from 35 to 65 mL for 100 g of nanocrystalline cellulose.

7. The sunscreen composition according to any of the preceding claims, wherein said functionalized nanocrystalline cellulose has an oil uptake of from 40 to 60 mL for 100 g of nanocrystalline cellulose.

8. The sunscreen composition according to any of the preceding claims, wherein said functionalized nanocrystalline cellulose is present in an amount of from 0.1% to 50%, relative to the total weight of the composition.

9. The sunscreen composition according to any of the preceding claims, wherein said functionalized nanocrystalline cellulose is present in an amount of from 0.2% to 30%, relative to the total weight of the composition.

10. The sunscreen composition according to any of the preceding claims, wherein said functionalized nanocrystalline cellulose is present in an amount of from 0.5% to 10% by weight, relative to the total weight of the composition

11. The sunscreen composition according to any of the preceding claims, wherein the at least one sunscreen agent is selected from mineral sunscreen agents and/or organic sunscreen agents.

12. The sunscreen composition according to any of the preceding claims, wherein the at least one mineral sunscreen agent is selected from zinc oxide and/or titanium dioxide.

13. The sunscreen composition according to any of the preceding claims, wherein said sunscreen composition comprises a water phase and an oily phase.

14. The sunscreen composition according to any of the preceding claims, wherein said sunscreen composition comprises at least one surfactant.

15. The sunscreen composition according to any of the claims 1 to 13, wherein the composition is surfactant-free.

16. The sunscreen composition according to claim 13, wherein said water phase comprises at least one humectant.

17. The sunscreen composition according to claim 13, wherein said oily phase comprises at least one emollient.

18. The sunscreen composition according to any of the preceding claims, wherein the sunscreen composition further comprises preservatives, cosmetic active ingredients, moisturizers, alcohol, and/or fragrances.

19. The sunscreen composition according to any of the preceding claims, wherein the sunscreen composition is in the form of a gel, a lotion, a serum.

## Patentansprüche

1. Sonnenschutzzusammensetzung, umfassend, in einem physiologisch akzeptablen Medium,
(i) mindestens ein Sonnenschutzmittel, und
(ii) eine funktionalisierte, nanokristalline Cellulose,
wobei die funktionalisierte, nanokristalline Cellulose carboxylierte, nanokristalline Cellulose ist,
wobei die funktionalisierte, nanokristalline Cellulose aus Nanokristalliten von Cellulose besteht, welche zu Partikeln von nanokristalliner Cellulose zusammengesetzt sind,
wobei die Partikel eine sphärische oder eiförmige Form aufweisen, und
wobei die funktionalisierte, nanokristalline Cellulose eine durchschnittliche Partikelgröße von 2 µm bis 10 µm aufweist.

2. Sonnenschutzzusammensetzung nach Anspruch 1, wobei die carboxylierte, nanokristalline Cellulose in der Form eines nanokristallinen Cellulosecarboxylatsalzes vorliegt.

3. Sonnenschutzzusammensetzung nach Anspruch 1, wobei das nanokristalline Cellulosecarboxylatsalz ein nanokristallines Cellulosenatriumcarboxylat ist.

4. Sonnenschutzzusammensetzung nach Anspruch 2, wobei das nanokristalline Cellulosenatriumcarboxylat durch das Verfahren erzeugt wird, umfassend die Schritte:
(a) Bereitstellen von Cellulose, und
(b) Mischen der Cellulose mit einem Peroxid, wodurch ein Reaktionsgemisch erzeugt wird, und
(c) Erhitzen des Reaktionsgemischs, und/oder
(c') Aussetzen der Reaktionsmischung gegenüber UV-Strahlung, und
(d) Überführen der Reaktionsmischung in ein Salz.

5. Sonnenschutzzusammensetzung nach einem der vorhergehenden Ansprüche, wobei die funktionalisierte, nanokristalline Cellulose eine Ölaufnahme von weniger als 70 ml pro 100 g nanokristalliner Cellulose aufweist.

6. Sonnenschutzzusammensetzung nach einem der vorhergehenden Ansprüche, wobei die funktionalisierte, nanokristalline Cellulose eine Ölaufnahme von 35 bis 65 ml pro 100 g nanokristalliner Cellulose aufweist.

7. Sonnenschutzzusammensetzung nach einem der vorhergehenden Ansprüche, wobei die funktionalisierte, nanokristalline Cellulose eine Ölaufnahme von 40 bis 60 ml pro 100 g nanokristalliner Cellulose aufweist.

8. Sonnenschutzzusammensetzung nach einem der vorhergehenden Ansprüche, wobei die funktionalisierte, nanokristalline Cellulose in einer Menge von 0,1 % bis 50 %, bezogen auf das Gesamtgewicht der Zusammensetzung, vorhanden ist.

9. Sonnenschutzzusammensetzung nach einem der vorhergehenden Ansprüche, wobei die funktionalisierte, nanokristalline Cellulose in einer Menge von 0,2 % bis 30 %, bezogen auf das Gesamtgewicht der Zusammensetzung, vorhanden ist.

10. Sonnenschutzzusammensetzung nach einem der vorhergehenden Ansprüche, wobei die funktionalisierte, nanokristalline Cellulose in einer Menge von 0,5 bis 10 Gew.-%, bezogen auf das Gesamtgewicht der Zusammensetzung, vorhanden ist.

11. Sonnenschutzzusammensetzung nach einem der vorhergehenden Ansprüche, wobei das mindestens eine Sonnenschutzmittel ausgewählt ist aus mineralischen Sonnenschutzmitteln und/oder organischen Sonnenschutzmitteln.

12. Sonnenschutzzusammensetzung nach einem der vorhergehenden Ansprüche, wobei das mindestens eine mineralische Sonnenschutzmittel ausgewählt ist aus Zinkoxid und/oder Titandioxid.

13. Sonnenschutzzusammensetzung nach einem der vorhergehenden Ansprüche, wobei die Sonnenschutzzusammensetzung eine Wasserphase und eine Ölphase umfasst.

14. Sonnenschutzzusammensetzung nach einem der vorhergehenden Ansprüche, wobei die Sonnenschutzzusammensetzung mindestens ein Tensid umfasst.

15. Sonnenschutzzusammensetzung nach einem der Ansprüche 1 bis 13, wobei die Zusammensetzung frei von Tensiden ist.

16. Sonnenschutzzusammensetzung nach Anspruch 13, wobei die Wasserphase mindestens ein Befeuchtungsmittel umfasst.

17. Sonnenschutzzusammensetzung nach Anspruch 13, wobei die Ölphase mindestens ein Emolliens umfasst.

18. Sonnenschutzzusammensetzung nach einem der vorhergehenden Ansprüche, wobei die Sonnenschutzzusammensetzung ferner Konservierungsmittel, kosmetische Wirkstoffe, Feuchtigkeitsspender, Alkohol und/oder Duftstoffe umfasst.

19. Sonnenschutzzusammensetzung nach einem der vorhergehenden Ansprüche, wobei die Sonnenschutzzusammensetzung in Form eines Gels, einer Lotion oder eines Serums vorliegt.

## Revendications

1. Composition d'écran scolaire comprenant, dans un milieu physiologiquement acceptable,
(i) au moins un agent d'écran solaire, et
(ii) une cellulose nanocristalline fonctionnalisée,
dans laquelle ladite cellulose nanocristalline fonctionnalisée est une cellulose nanocristalline carboxylée,
dans laquelle ladite cellulose nanocristalline fonctionnalisée est composée de nanocristallites de cellulose assemblés en particules de cellulose nanocristalline,
dans laquelle lesdites particules présentent une forme sphérique ou ovoïde, et
dans laquelle ladite cellulose nanocristalline fonctionnalisée présente une taille de particule moyenne de 2 µm à 10 µm.

2. Composition d'écran solaire selon la revendication 1 dans laquelle ladite cellulose nanocristalline carboxylée est sous forme d'un sel de carboxylate de cellulose nanocristalline.

3. Composition d'écran solaire selon la revendication 1 dans laquelle ledit sel de carboxylate de cellulose nanocristalline est un carboxylate de sodium de cellulose nanocristalline.

4. Composition d'écran solaire selon la revendication 2 dans laquelle ledit carboxylate de sodium de cellulose nanocristalline est produit par le procédé comprenant les étapes de :
(a) fourniture d'une cellulose, et
(b) mélange de ladite cellulose avec un peroxyde, produisant ainsi un mélange réactionnel, et
(c) chauffage du mélange réactionnel, et/ou
(c') exposition du mélange réactionnel à un rayonnement UV, et
(d) salification du mélange réactionnel.

5. Composition d'écran solaire selon l'une quelconque des revendications précédentes, dans laquelle ladite cellulose nanocristalline fonctionnalisée présente une prise d'huile inférieure à 70 mL pour 100 g de cellulose nanocristalline.

6. Composition d'écran solaire selon l'une quelconque des revendications précédentes, dans laquelle ladite cellulose nanocristalline fonctionnalisée présente une prise d'huile de 35 à 65 mL pour 100 g de cellulose nanocristalline.

7. Composition d'écran solaire selon l'une quelconque des revendications précédentes, dans laquelle ladite cellulose nanocristalline fonctionnalisée présente une prise d'huile de 40 à 60 mL pour 100 g de cellulose nanocristalline.

8. Composition d'écran solaire selon l'une quelconque des revendications précédentes, dans laquelle ladite cellulose nanocristalline fonctionnalisée est présente dans une quantité de 0,1 % à 50 %, par rapport au poids total de la composition.

9. Composition d'écran solaire selon l'une quelconque des revendications précédentes, dans laquelle ladite cellulose nanocristalline fonctionnalisée est présente dans une quantité de 0,2 % à 30 %, par rapport au poids total de la composition.

10. Composition d'écran solaire selon l'une quelconque des revendications précédentes, dans laquelle ladite cellulose nanocristalline fonctionnalisée est présente dans une quantité de 0,5 % à 10 % en poids, par rapport au poids total de la composition.

11. Composition d'écran solaire selon l'une quelconque des revendications précédentes, dans laquelle l'au moins un agent d'écran solaire est choisi parmi des agents d'écran solaire minéraux et/ou des agents d'écran solaire organiques.

12. Composition d'écran solaire selon l'une quelconque des revendications précédentes, dans laquelle l'au moins un agent d'écran solaire minéral est choisi parmi l'oxyde de zinc et/ou le dioxyde de titane.

13. Composition d'écran solaire selon l'une quelconque des revendications précédentes, dans laquelle ladite composition d'écran solaire comprend une phase aqueuse et une phase huileuse.

14. Composition d'écran solaire selon l'une quelconque des revendications précédentes, dans laquelle ladite composition d'écran solaire comprend au moins un tensio-actif.

15. Composition d'écran solaire selon l'une quelconque des revendications 1 à 13, dans laquelle la composition est exempte de tensio-actif.

16. Composition d'écran solaire selon la revendication 13, dans laquelle ladite phase aqueuse comprend au moins un humectant.

17. Composition d'écran solaire selon la revendication 13, dans laquelle ladite phase huileuse comprend au moins un émollient.

18. Composition d'écran solaire selon l'une quelconque des revendications précédentes, dans laquelle la composition d'écran solaire comprend en outre des conservateurs, des principes actifs cosmétiques, des hydratants, de l'alcool, et/ou des parfums.

19. Composition d'écran solaire selon l'une quelconque des revendications précédentes, dans laquelle la composition d'écran solaire est sous forme d'un gel, d'une lotion, d'un sérum.
